# EUROPEAN PATENT SPECIFICATION

(11) **EP 4 382 084 B1**
(45) Date of publication and mention of the grant of the patent: **20.11.2024**
(21) Application number: 22211293.0
(22) Date of filing: 05.12.2022
(51) Int. Cl.: A61F 15/00, B65D 83/08

(54) **PLASTER DISPENSER**
PFLASTERSPENDER
DISTRIBUTEUR DE PANSEMENTS

(43) Date of publication of application: 12.06.2024
(73) Proprietor: Plum Safety ApS, 5610 Assens (DK)
(72) Inventor: Barkholt, Bo Winther, Assens (DK)
(74) Representative: RGTH

(56) References cited:
- EP-A1- 1 642 558
- US-A- 4 194 624
- US-B2- 7 644 818

## Description

The present invention relates to a plaster dispenser as well as to a system of a plaster dispenser and a plaster pack according to the independent claims.

### Prior art

Plaster dispensers are generally known from the prior art. For example, US D650210 S1 describes a plaster dispenser with a front area and two sidewalls defining a space in which a plaster pack can be placed. However, empty plaster packs can only be removed with quite some effort, while the plaster dispenser is further complicated to produce.

US 7,644,818 B2 A discloses a plaster dispenser for moving plasters individually from a plaster pack including several plasters.

US 4,194,624 A relates to a storage receptable for multiple adhesive bandage pack including front and rear walls joined at their lower extremities.

In EP 1 642 558 A1 a plaster dispenser is disclosed which is constructed as a box fastened to a wall.

### Present invention

The objective of the present invention is to provide a plaster dispenser such that its production is cost effective and particularly easy, while further exchanging plaster packs is made as effortless as possible.

The objective is solved by a plaster dispenser for removing plasters individually from a plaster pack, wherein the dispenser has an inner space for accommodating the plaster pack. According to the invention, the dispenser consists of two walls being a front wall and a rear wall creating the inner space for accommodating the plaster pack. In other words, the dispenser only consists of the front wall and the rear wall. The dispenser does not comprise side walls for confining the inner space. The inner space is thus confined in its thickness direction by the front wall and the rear wall, while it is open to both sides in its width direction. This results in the great advantage that an empty plaster pack can easily be removed to the side.

The dispenser is also open to the lower side such as to insert a new plaster pack. Since gravity acts downwardly, the plaster pack is securely held by the dispenser. To remove an empty plaster pack, it can be pushed to the upper side and then swiped to the left or to the right side where the dispenser is configured open and thus be removed. Since the dispenser is only configured of the front and the rear wall, it can be constructed easily in only one injection molding step which significantly improves its manufacturing. The entire plaster dispenser is advantageously made of one piece and one material, e.g., from plastic, which considerably makes its production easy and cost effective.

The front wall can be slightly curved while the rear wall can be configured plane. Especially, the rear wall is shorter than the front wall. In particular, the length of the rear wall is less than 70 %, more preferred less than 60 %, of a length of the front wall.

The front wall and the rear wall can enclose an angle which can be between 10° and 30°, preferably between 15° and 20°. If the front wall is curved the angle can be still determined by placing an imaginary line between the ends of the respective wall in length direction and determining the angle between those.

According to the invention, the free edge of the rear wall has an inwardly projecting rear shoulder projecting into the inner shape. The rear shoulder can further project outwardly such that when the dispenser is fastened at a wall, particular at an upper or middle part of the dispenser, the rear wall is at an angle to the wall. The angle is preferably between 2° and 20°, most preferred between 5° and 10°.

The rear wall with its rear shoulder at its free edge can have a T-shape in cross section of the dispenser. The rear shoulder can in a cross section of the dispenser be at a right angle to the rear wall. The cross section can be in thickness direction.

Particular, a part of the rear shoulder projects outwardly while another part projects inwardly. Especially, the part projecting outwardly is longer than the part projecting inwardly. In other words, the rear shoulder projects further outwardly than inwardly.

Especially the length of the part of the rear shoulder projecting outwardly in a cross section of the dispenser is at least twice as long as the part of the rear shoulder projecting inwardly.

According to the invention, the free edge of the front wall has an inwardly protruding front shoulder projecting into the inner space. Further, the front shoulder does preferably not protrude outwardly. The front wall with its front shoulder at its free edge can have an L-shape in cross section of the dispenser. The front shoulder in a cross section of the dispenser can be at a right angle to the front wall. According to the invention, a first edge of the plaster pack is engageable with the rear shoulder of the plaster dispenser, while a second edge of the plaster pack is engageable with the front shoulder of the plaster dispenser such that the plaster pack is securely held.

The length of the part of the rear shoulder protruding inwardly is at least a tenth, more particular at least an eighth of the length of the rear wall. The length is at most a quarter of the length of the rear wall.

The length of the front shoulder protruding inwardly especially in a cross section of the dispenser is at most one eighth of the length of the front shoulder and/or at least one thirtieth of its length.

The front wall as well as the rear wall each have at least one opening for fastening means for fastening the dispenser on the wall. Especially, the front wall has an opening for each opening of the rear wall. These openings are aligned such that the dispenser can be fastened on a wall for example by fastening means, such as screws. Especially, the front wall and the rear wall can each have two openings.

The rear wall can have a circumferential projection around each opening protruding outwardly for ensuring that the rear wall is at an angle to a wall at which the dispenser can be fastened. Furthermore, the circumferential projection can serve for guiding the fastening means which serve to fasten the dispenser at the wall.

The front wall can have a recess for providing improved access to the inner space and thus to the plaster pack. The recess is preferably arranged in the middle of the front wall in its width direction. It can extend at least along a seventh of the width of the front wall. Especially, the width of the front wall and the rear wall is the same.

Furthermore, the rear front can have a recess. The recess is preferably arranged in the middle of the rear wall in its width direction. The recess can be larger than the recess of the front wall. It can extend at least along a quarter of the width of the rear wall.

The recess of the front wall and/or the rear wall can be disposed such that it divides the free end of the respective wall as well as the respective shoulder into two parts. The shoulder is not disposed in the area in which the recess is disposed. The rear shoulder and/or the front shoulder extends continuously along at least 60 %, preferably along 70 %, most preferred along 90 % of the width of the dispenser, except for the recess.

In a further aspect of the invention, it relates to a system of a plaster dispenser and a plaster pack. The plaster dispenser is configured according to any of the features as described above.

The plaster pack is preferably essentially box-shaped. It is configured to at least in part fit into the inner space of the dispenser. The plaster pack has a first edge on a first side to engage with the rear shoulder. Alternatively or additionally, the plaster pack can have a second edge on a second side to engage with the front shoulder of the plaster dispenser. The two sides of the plaster pack are preferably at different sides of the plaster pack in thickness direction.

The first edge is engageable with the rear shoulder of the plaster dispenser, while the second edge is engageable with the front shoulder of the plaster dispenser. In this way the plaster pack is held such that it does not fall out of the dispenser. An engagement can mean that the at least one edge lies on at least one shoulder. Furthermore, both edges can lie on a shoulder respectively. This secures the plaster pack in the inner space despite of the fact that the inner space is open to the downward direction and thus the direction of gravity. In other words, the engagement prevents the plaster pack from falling out of the dispenser. The plaster pack, especially when empty, however, can still be lifted to the upper direction, thus against gravity, such that it is unengaged and can be slid to the side.

The first and/or the second edge can be configured as a wall of a protrusion or an intrusion into the plaster pack. Further, the plaster pack can have a constriction which creates the edge.

### Brief description of the figures

In schematic representation, the figures show:
- Figure 1:: a perspective representation of a plaster dispenser;
- Figure 2:: a side view of the plaster dispenser of figure 1;
- Figure 3:: a side view from below onto the dispenser of figures 1 and 2; and
- Figure 4:: a system between a plaster dispenser and a plaster pack.

### Preferred embodiments

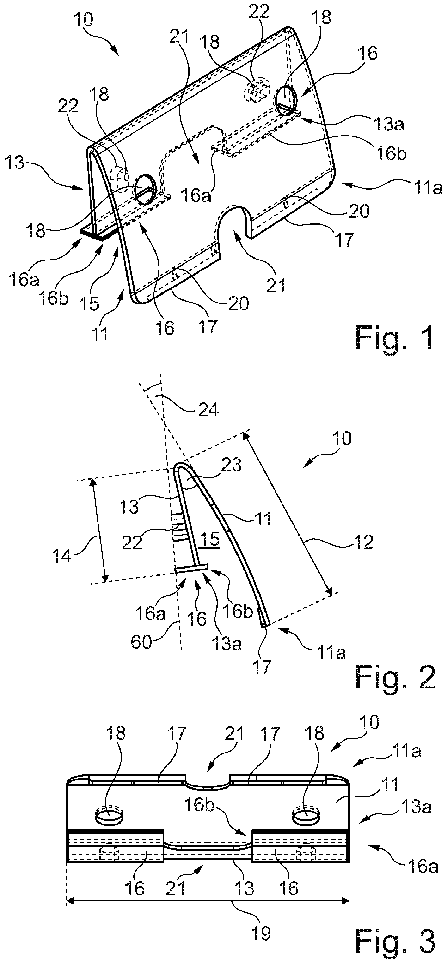

Figure 1 shows a perspective representation of a plaster dispenser 10 which has a front wall 11 and a rear wall 13, wherein the rear wall 13 is considerably shorter than the front wall 11.

The front wall 11 as well as the rear wall 13 have two ends in their respective length direction, at one end of which they are connected to each other, as well as a respective free end. The front wall 11 has a free end 11a and the rear wall 13 has a free end 13a. At the free end 11a of the front wall, 11 the front shoulder 17 is disposed, while at the free end 13a of the rear wall 13, the rear shoulder 16 is disposed.

Between the front wall 11 and the rear wall 13, an inner space 15 is formed which is open to the downward side in figure 1 as well as to both sides of the dispenser 10, since the plaster dispenser 10 does not have side walls. The front wall 11 has a front shoulder 17 at its edge, in other words at its free end 11a.

The front wall 11 has a recess for an improved access to the inner space 15 which can accommodate a plaster pack 50. The front shoulder 17 is divided by the recess 21 into two parts. The rear wall 13 also has a recess 21 which divides the rear shoulder 16 into two parts.

The front wall 11 as well as the rear wall 13 have openings 18 for allowing fastening means to fasten the plaster dispenser 10 at a wall. The front wall 11 can have reinforcement bars 20 reinforcing the rear shoulder 16.

In figure 2 a side view of the plaster dispenser 10 of figure 1 is shown. Since there is no side wall, the inner space 15 can be well seen. Furthermore, the front wall 11 which is slightly curved and the rear wall 13 can be seen which at its free ends, namely at its edges, have the front shoulder 17 and the rear shoulder 16.

The front shoulder 17 protrudes inwardly into the inner space 15, while the rear shoulder 16 protrudes inwardly as well as outwardly. The rear shoulder 16 forms a T of the edge of the rear wall 13. It has a first part 16a protruding outwardly and a second part 16b protruding inwardly. The first part 16a is protruding farther outwardly than the second part 16b is protruding inwardly.

Furthermore, around the openings 18 in the rear wall 13, circumferential projections 22 protrude outwardly (see also figure 1). Furthermore, in figure 2 it can be seen that the length 12 of the front wall 11 is far greater than the length 14 of the rear wall 13. In addition, it can be seen that the two walls have an angle 23 which they enclose.

Figure 2 shows an imaginary wall 60 to which the dispenser 10 can be fastened. The rear wall 13 and the wall 60 have an angle 24 which they enclose.

In figure 3 a side view from below onto the dispenser of figures 1 and 2 is shown. The front shoulder 17 as well as rear shoulder 16 can be viewed easily as well as the recesses 21 which divide the respective shoulders into two parts. Further, the openings 18 in the front wall 11 can well be seen. Further, figure 3 shows the width 19 of both the front wall 11 and the rear wall 13.

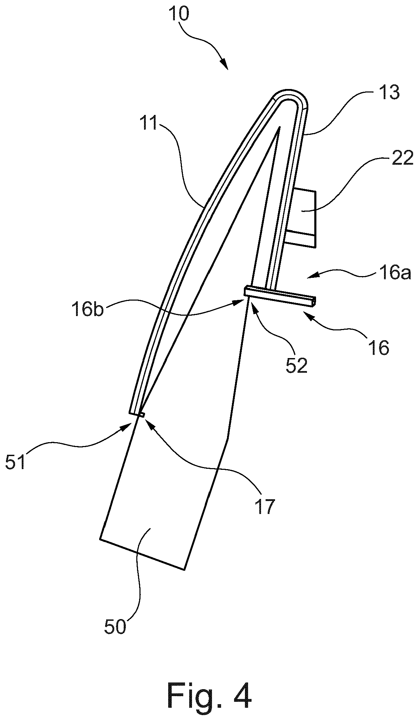

Figure 4 shows a system between a plaster dispenser 10 and a plaster pack 50. The plaster pack 50 protrudes into the inner space 18 between the front wall 11 and the rear wall 13 of the plaster dispenser 10, which is configured according to figures 1 to 3.

The plaster pack 50 has a first edge 51 on a first side and a second edge 52 on a second side. The first edge 51 is configured to engage with the front shoulder 17 of the plaster dispenser 10, while the second edge 52 is engaged with the rear shoulder 16, in particular with its inwardly protruding part 16b. In detail, due to the edges, the plaster pack can securely held in the inner space, despite the fact that the inner space 15 is open to the downward direction and thus in the direction of gravity.

### Reference signs

- 10: plaster dispenser
- 11: front wall
- 11a: free edge of front wall
- 12: length of front wall
- 13: rear wall
- 13a: free edge of rear wall
- 14: length of rear wall
- 15: inner space
- 16: rear shoulder
- 16a: outwardly protruding part
- 16b: inwardly protruding part
- 17: front shoulder
- 18: opening
- 19: width
- 20: reinforcement bar
- 21: recess
- 22: circumferential projection
- 23: angle between front and rear wall
- 24: angle between rear wall and wall

- 50: plaster pack
- 51: first edge
- 52: second edge
- 60: wall

## Claims

1. A plaster dispenser (10) for removing plasters individually from a plaster pack (50),
wherein the dispenser (10) has an inner space (15) for accommodating the plaster pack (50),
wherein
the dispenser (10) consists of two walls being a front wall (11) and a rear wall (13) creating the inner space (15) for the plaster pack (50).
**characterized in that**
the free edge (13a) of the rear wall (13) has an inwardly protruding rear shoulder (16) projecting into the inner space (15),
wherein the free edge (11a) of the front wall (11) has an inwardly protruding front shoulder (17) projecting into the inner space (15), and
wherein the rear shoulder (16) is engageable with a first edge of the plaster pack (50) and the front shoulder (17) is engageable with a second edge of the plaster pack (50) such that the plaster pack (50) is securely held.

2. The plaster dispenser (10) according to claim 1,
**characterized in that**
the rear wall (13) is shorter than the front wall (11).

3. The plaster dispenser (10) according to claim 1 or 2,
**characterized in that**
a length (14) of the rear wall (13) is less than 70 percent of a length (12) of the front wall (11).

4. The plaster dispenser (10) according to claim 1,
**characterized in that**
the rear shoulder (16) further projects outwardly such that when the dispenser (10) is fastened at a wall (60), the rear wall (13) is at an angle (24) to the wall (60).

5. The plaster dispenser (10) according to claim 1 or 4,
**characterized in that**
a part (16a) of the rear shoulder (16) projecting outwardly is longer than a part (16b) of the rear shoulder (16) projecting inwardly.

6. The plaster dispenser (10) according to claim 1,
**characterized in that**
the front shoulder (17) protrudes perpendicular to the front wall (11) and/or the rear shoulder (16) protrudes perpendicular to the rear wall (13).

7. The plaster dispenser (10) according to any one of the claims 1, , 5 or 6,
**characterized in that**
the front wall (11) and the rear wall (13) have at least one opening (18) for fastening the plaster dispenser (10) on a wall (60).

8. The plaster dispenser (10) according to claim 7,
**characterized in that**
the rear wall (13) has a circumferential projection (22) around each opening (18).

9. The plaster dispenser (10) according to any one of the preceding claims, **characterized in that**
the front wall (11) and/or the rear wall (13) has a recess (21).

10. A system of a plaster dispenser (10) and a plaster pack (50),
**characterized in that**
the plaster dispenser (10) is configured according to one of the claims 1 to 9.

11. The system according to claim 10,
**characterized in that**
the plaster pack (50) has a first edge (51) on one side to engage with the front shoulder (17) of the plaster dispenser (10) and/or a second edge (52) on another side to engage with the rear shoulder (16) of the plaster dispenser (10).

## Patentansprüche

1. Pflasterspender (10) zur individuellen Entnahme von Pflastern aus einer Pflasterpackung (50),
wobei der Spender (10) einen Innenraum (15) zur Aufnahme der Pflasterpackung (50) aufweist,
wobei
der Spender (10) aus zwei Wänden besteht, nämlich einer Vorderwand (11) und einer Rückwand (13), die den Innenraum (15) für die Pflasterpackung (50) bilden.
**dadurch gekennzeichnet, dass**
die freie Kante (13a) der Rückwand (13) eine nach innen vorspringende hintere Schulter (16) aufweist, die in den Innenraum (15) hineinragt,
wobei die freie Kante (11a) der Vorderwand (11) eine nach innen vorspringende vordere Schulter (17) aufweist, die in den Innenraum (15) hineinragt, und
wobei die hintere Schulter (16) mit einer ersten Kante der Pflasterpackung (50) in Eingriff bringbar ist und die vordere Schulter (17) mit einer zweiten Kante der Pflasterpackung (50) in Eingriff bringbar ist, so dass die Pflasterpackung (50) sicher gehalten wird.

2. Pflasterspender (10) gemäß Anspruch 1,
**dadurch gekennzeichnet, dass**
die Rückwand (13) kürzer als die Vorderwand (11) ist.

3. Pflasterspender (10) gemäß Anspruch 1 oder 2,
**dadurch gekennzeichnet, dass**
eine Länge (14) der Rückwand (13) weniger als 70 Prozent einer Länge (12) der Vorderwand (11) beträgt.

4. Pflasterspender (10) gemäß Anspruch 1,
**dadurch gekennzeichnet, dass**
die hintere Schulter (16) weiter nach außen ragt, so dass, wenn der Spender (10) an einer Wand (60) befestigt ist, die Rückwand (13) in einem Winkel (24) zu der Wand (60) steht.

5. Pflasterspender (10) gemäß Anspruch 1 oder 4,
**dadurch gekennzeichnet, dass**
ein nach außen vorstehender Teil (16a) der hinteren Schulter (16) länger ist als ein nach innen vorstehender Teil (16b) der hinteren Schulter (16).

6. Pflasterspender (10) gemäß Anspruch 1,
**dadurch gekennzeichnet, dass**
die vordere Schulter (17) rechtwinklig zur Vorderwand (11) vorspringt und/oder die hintere Schulter (16) rechtwinklig zur Rückwand (13) vorspringt.

7. Pflasterspender (10) gemäß einem der Ansprüche 1, 5 oder 6,
**dadurch gekennzeichnet, dass**
die Vorderwand (11) und die Rückwand (13) mindestens eine Öffnung (18) zur Befestigung des Pflasterspenders (10) an einer Wand (60) aufweisen.

8. Pflasterspender (10) gemnäß Anspruch 7,
**dadurch gekennzeichnet, dass**
die Rückwand (13) um jede Öffnung (18) herum einen umlaufenden Vorsprung (22) aufweist.

9. Pflasterspender (10) gemäß einem der vorhergehenden Ansprüche,
**dadurch gekennzeichnet, dass**
die Vorderwand (11) und/oder die Rückwand (13) eine Aussparung (21) aufweist.

10. System aus einem Pflasterspender (10) und einer Pflasterpackung (50),
**dadurch gekennzeichnet, dass**
der Pflasterspender (10) gemäß einem der Ansprüche 1 bis 9 konfiguriert ist.

11. System gemäß Anspruch 10,
**dadurch gekennzeichnet, dass**
die Pflasterpackung (50) eine erste Kante (51) auf einer Seite aufweist, um mit der vorderen Schulter (17) des Pflasterspenders (10) in Eingriff zu kommen und/oder eine zweite Kante (52) auf einer anderen Seite, um mit der hinteren Schulter (16) des Pflasterspenders (10) in Eingriff zu kommen.

## Revendications

1. Distributeur de sparadrap (10) pour retirer des sparadraps individuellement d'un paquet de sparadraps (50),
dans lequel le distributeur (10) a un espace intérieur (15) pour loger le paquet de sparadraps (50),
dans lequel
le distributeur (10) est constitué de deux parois, une paroi avant (11) et une paroi arrière (13) créant l'espace intérieur (15) pour le paquet de sparadraps (50),
**caractérisé par le fait que**
le bord libre (13a) de la paroi arrière (13) comporte un épaulement arrière (16) faisant saillie vers l'intérieur et s'avançant dans l'espace intérieur (15),
dans lequel le bord libre (11a) de la paroi avant (11) présente un épaulement avant (17) faisant saillie vers l'intérieur et s'avançant dans l'espace intérieur (15), et
dans lequel l'épaule arrière (16) est en prise avec un premier bord du paquet de sparadraps (50) et l'épaule avant (17) est en prise avec un second bord du paquet de sparadraps (50) de manière à ce que le paquet de sparadraps (50) soit solidement maintenue.

2. Distributeur de sparadrap (10) selon revendication 1,
**caractérisé par le fait que**
la paroi arrière (13) est plus courte que la paroi avant (11).

3. Distributeur de sparadrap (10) selon la revendication 1 ou 2,
**caractérisé par le fait que**
une longueur (14) de la paroi arrière (13) est inférieure à 70 % d'une longueur (12) de la paroi avant (11).

4. Distributeur de sparadrap (10) selon la revendication 1,
**caractérisé par le fait que**
l'épaulement arrière (16) fait en outre saillie vers l'extérieur de sorte que lorsque le distributeur (10) est fixé à une paroi (60), la paroi arrière (13) forme un angle (24) avec la paroi (60).

5. Distributeur de sparadrap (10) selon la revendication 1 ou 4,
**caractérisé par le fait que**
une partie (16a) de l'épaule arrière (16) faisant saillie vers l'extérieur est plus longue qu'une partie (16b) de l'épaule arrière (16) faisant saillie vers l'intérieur.

6. Distributeur de sparadrap (10) selon la revendication 1,
**caractérisé par le fait que**
l'épaulement avant (17) fait saillie perpendiculairement à la paroi avant (11) et/ou l'épaulement arrière (16) fait saillie perpendiculairement à la paroi arrière (13).

7. Distributeur de sparadrap (10) selon l'une quelconque des revendications 1, 5 ou 6,
**caractérisé par le fait que**
la paroi avant (11) et la paroi arrière (13) ont au moins une ouverture (18) pour fixer le distributeur de sparadrap (10) à une paroi (60).

8. Distributeur de sparadrap (10) selon la revendication 7,
**caractérisé par le fait que**
la paroi arrière (13) présente une saillie circonférentielle (22) autour de chaque ouverture (18).

9. Distributeur de sparadrap (10) selon l'une quelconque des revendications précédentes,
**caractérisé par le fait que**
la paroi avant (11) et/ou la paroi arrière (13) comporte une cavité (21).

10. Système composé d'un distributeur de sparadrap (10) et d'un paquet de sparadraps (50),
**caractérisé par le fait que**
le distributeur de sparadrap (10) est configuré selon l'une des revendications 1 à 9.

11. Le système selon la revendication 10,
**caractérisé par le fait que**
le paquet de sparadraps (50) a un premier bord (51) d'un côté pour s'engager dans l'épaule avant (17) du distributeur de sparadrap (10) et/ou un deuxième bord (52) d'un autre côté pour s'engager dans l'épaule arrière (16) du distributeur de sparadrap (10).
